# DEMANDE DE BREVET EUROPEEN

(11) **EP 1 430 869 A1**
(43) Date de publication de la demande: **23.06.2004**
(21) Numéro de dépôt: 03292895.4
(22) Date de dépôt: 21.11.2003
(51) Int. Cl.: A61K 7/06, A61K 7/50

(54) **Composition de lavage des matières kératiniques à base alkyléthersulfate et de tensioactif amphotère**

(30) Priorité: 19.12.2002 FR 0216195
(71) Demandeur: L'OREAL, 75008 Paris (FR)
(72) Inventeur: Gawtrey, Jonathan, 92100 Boulogne (FR); Maubru, Mireille, 78400 Chatou (FR)
(74) Mandataire: Le Blainvaux Bellegarde, Françoise

(57) **Abrégé**

La présente invention est relative à des compositions de lavage des matières kératiniques, et en particulier des cheveux et/ou de la peau, comprenant au moins un tensio-actif anionique alkyléthersulfate et au moins 2,5% d'un tensioactif amphotère alcoylamphohydroxyalkylsulfonate, le rapport en poids du tensioactif alkyléthersulfate et du tensioactif amphotère sulfonate étant de 2 à 20 et au procédé de lavage mettant en oeuvre ces compositions.

## Description

La présente invention est relative à des compositions de lavage des matières kératiniques, et en particulier des cheveux et/ou de la peau, comprenant au moins un tensioactif anionique alkyléthersulfate et au moins un tensioactif amphotère alcoylamphohydroxyalkylsulfonate et au procédé de lavage mettant en oeuvre ces compositions.

Il est connu d'utiliser des tensio-actifs amphotères, tels que les alkylimidazolines ou des bétaïnes dans des shampooings. Ces tensioactifs font office de détergents auxiliaires dans ce type de formulation.

Les tensioactifs amphotères alcoylamphohydroxyalkylsulfonates ont déjà été préconisés dans des compositions cosmétiques détergentes. Ils ont été décrits par exemple dans la demande de brevet WO99/36054.

Toutes ces compositions de l'art antérieur présentent la caractéristique de ne pas donner totalement satisfaction en particulier de ne pas avoir de propriétés cosmétiques suffisantes, notamment le démêlage et le lissage des cheveux séchés.

La demanderesse a découvert, de façon surprenante, qu'il était possible de formuler des compositions de lavage des matières kératiniques, notamment des shampooings en utilisant un tensioactif amphotère particulier en association avec un tensioactif alkyléther sulfate dans des proportions particulières.

L'invention a pour objet des compositions de lavage des matières kératiniques comprenant au moins un tensio-actif anionique alkyléthersulfate et au moins 2,5% d'un tensioactif amphotère alcoylamphohydroxyalkylsulfonate, le rapport en poids tensioactif alkyléthersulfate / tensioactif amphotère sulfonate allant de 2 à 20.

L'invention a également pour objet l'utilisation d'au moins 2,5% en poids d'un tensioactif amphotère alcoylamphohydroxyalkylsulfonate dans le but d'améliorer le démêlage et le lissage des compositions de lavage des matières kératiniques contenant un tensio-actif anionique alkyléthersulfate.

Un autre objet de l'invention est un procédé de lavage des matières kératiniques.

D'autres objets de l'invention apparaîtront à la lecture de la description et des exemples qui suivent.

Selon la présente invention, par matières kératiniques, on comprend les cheveux, les cils, les sourcils, la peau, les ongles, les muqueuses ou le cuir chevelu et plus particulièrement les cheveux.

Les différents objets de l'invention vont maintenant être détaillés. L'ensemble des significations et définitions des composés utilisés dans la présente invention données ci-dessous sont valables pour l'ensemble des objets de l'invention.

Les tensioactifs amphotères alcoylamphohydroxyalkylsulfonates peuvent avoir la formule suivante (I) : 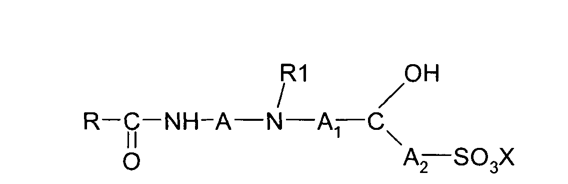 dans laquelle :
R désigne un radical hydrocarboné, saturé ou insaturé, linéaire ou ramifié comportant de 5 à 29 atomes de carbone.
R désigne de préférence un radical alkyle ou alkényle mono ou polyinsaturé comportant de 5 à 29 atomes de carbone et de préférence de 7 à 22 atomes de carbone et encore plus particulièrement de 9 à 17 atomes de carbone.
R1 désigne un radical hydroxyalkyle en C₁-C_{4,} de préférence hydroxyéthyle,
A, A1, A2 , identiques ou différents, désignent un radical alkylène divalent linéaire ou ramifié en C₁-C₁₀, de préférence en C₁-C₃
X désigne un atome d'hydrogène, un cation minéral ou organique tel que:
   celui d'un métal alcalin (par exemple Na⁺, K⁺) , celui d'un métal alcalinoterreux (Mg²⁺, Ca²⁺) , l'ion NH₄⁺ , les ions ammoniums issus des aminoacides basiques ou des amino-alcools.

Des composés préférés selon la présente invention sont des composés de formule (II) dans laquelle R désigne plus particulièrement un radical alkyle saturé, linéaire ou ramifié comportant de 7 à 29 atomes de carbone, de préférence de 7 à 22 atomes de carbone.

Lorsque X désigne un ion ammonium issu d'alcanolamine, celle-ci peut être la monoéthanolamine, la diéthanolamine, la triéthanolamine, l'amino-3 propanediol-1,2. Lorsque X désigne un ion ammonium issu d'amine, cette amine peut être un aminoacide basique tel que la lysine, l'arginine, la sarcosine, l'ornithine ou la citrulline.

De préférence, A= A2 et désigne -CH₂CH₂-.

De préférence, A1 désigne -CH₂-

De préférence X désigne Na⁺

Parmi les tensioactifs de formule (I), on peut citer plus particulièrement les sels de Cocoyl amphohydroxypropyl sulfonate et notamment le sel de sodium tel que le produit proposé sous la dénomination MIRANOL CSE par la société RHODIA CHIMIE ou les sels de palmitoyl amphohydroxypropyl sulfonate et notamment le sel de sodium tel que le produit proposé sous la dénomination MIRANOL CS par la société RHODIA CHIMIE .

Selon l'invention, le ou les tensioactifs amphotères choisis parmi les composés alcoylamphohydroxyalkylsulfonates et leurs sels peuvent représenter de 2,5 % à 30 % en poids, de préférence de 3 % à 15 % en poids par rapport au poids total de la composition finale.

Les tensioactifs anioniques alkyl éthersulfate utilisables, seuls ou mélanges, dans le cadre de la présente invention, sont les sels (en particulier sels alcalins, notamment de sodium, sels d'ammonium, sels d'amines, sels d'aminoalcools ou sels de magnésium) des alkyléthersulfates, alkylamidoéthersulfates, alkylaryléthersulfates; le radical alkyle de tous ces différents composés comportant de préférence de 8 à 24 atomes de carbone, et le radical aryle désignant de préférence un groupement phényle ou benzyle.

Le nombre moyen de groupements oxyde d'éthylène ou oxyde de propylène peut aller notamment de 2 à 50 et plus particulièrement de 2 à 10.

Parmi ces tensioactifs anioniques, on préfère utiliser les sels d'alkyléthersulfates en C₈-C₁₄ et plus particulièrement ceux en C₁₂-C₁₄. Ces sels comprennent de préférence de 2 à 5 groupements d'oxyde d'éthylène.

Les tensioactifs anioniques alkyléthersulfates sont généralement présents à raison de 2,5 % à 50 % en poids, de préférence de 5 à 20 % en poids, par rapport au poids total de la composition.

Le rapport en poids tensioactif alkyléthersulfate / tensioactif amphotère sulfonate va de préférence de 2 à 10 et encore plus préférentiellement de 3 à 7.

La composition de la présente invention contient en outre un ou plusieurs tensioactifs additionnels choisis parmi les tensio-actifs non-ioniques, amphotères ou zwitterioniques différents des tensioactifs amphotères sus-définis.

Le ou les tensioactifs additionnels sont présents dans des quantités allant de 0,5 à 15% en poids par rapport au poids total de la composition.

La quantité totale de tensioactifs dans les compositions selon l'invention va de préférence de 5 à 55% en poids et de préférence de 7 à 30% en poids par rapport au poids total de la composition.

Les agents tensio-actifs non-ioniques sont plus particulièrement choisis parmi les alcools ou les alkylphénols ou les acides gras polyéthoxylés, polypropoxylés ou polyglycérolés, à chaîne grasse comportant de 8 à 20 atomes de carbone, le nombre de groupements oxyde d'éthylène ou oxyde de propylène étant compris entre 2 et 50 et le nombre de groupements glycérol étant compris entre 2 et 30, les esters de sorbitan d'acide gras en C₈-C₃₀ oxyéthyléné avec un nombre de moles d'oxyde d'éthylène compris entre 2 et 30.

On peut également citer les copolymères d'oxydes d'éthylène et de propylène; les condensats d'oxydes d'éthylène et de propylène sur des alcools gras; les amides gras polyéthoxylés ayant de préférence 2 à 30 moles d'oxyde d'éthylène; les amides gras polyglycérolés comportant de préférence 1 à 5 groupements glycérol et en particulier 1,5 à 4; les esters d'acides gras du sorbitan oxyéthylénés avec 12 à 30 moles d'oxyde d'éthylène; les esters d'acide gras de sucrose, les esters d'acides gras du polyéthylèneglycol, les alkylpolyglycosides, les dérivés carbamates ou amides de N-alkyl glucamines, les aldobionamides, les oxydes d'amines tels que les oxydes d'alkylamines ou de N-acylamidopropyl-morpholine.

Les compositions peuvent également contenir des agents tensio-actifs cationiques utilisés de préférence dans des proportions comprises entre 0,001 à 5% en poids, par rapport au poids total de la composition.

Les agents tensio-actifs cationiques sont notamment choisis parmi les sels d'amines grasses primaires, secondaires ou tertiaires, éventuellement polyoxyalkylénées; les sels d'ammonium quaternaire; les dérivés d'imidazoline; ou les oxydes d'amines à caractère cationique.

Les sels d'ammonium quaternaire préférés sont les halogénures (par exemple chlorures) de tétraalkylammonium comme, par exemple, les chlorures de dialkyldiméthylammonium ou d'alkyltriméthylammonium, dans lesquels le radical alkyle comporte environ de 12 à 22 atomes de carbone, en particulier les chlorures de béhényltriméthylammonium, de distéaryldiméthylammonium, de cétyltriméthylammonium, de benzyl diméthyl stéaryl ammonium ou encore le chlorure de stéaramidopropyl diméthyl (myristyl acétate) ammonium, commercialisés sous la dénomination de "CEPHARYL 70" par la Société VAN DYK.

On peut également utiliser les sels (chlorures ou méthylsulfate, notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium, et leurs mélanges.

Les radicaux acyle proviennent plus particulièrement d'une huile végétale comme l'huile de palme ou de tournesol.

La concentration des tensio-actifs additionnels autres que les tensio-actifs anioniques, peut varier de 0 à 30% et de préférence de 0,5 à 15% en poids, par rapport au poids total de la composition.

Les compositions selon l'invention peuvent contenir également des silicones, à savoir des huiles de polyorganosiloxanes, ou des gommes ou des résines de polyorganosiloxanes, telles que sous la forme de solutions dans des solvants organiques ou encore sous forme d'émulsions ou de microémulsions.

Parmi les polyorganosiloxanes pouvant être utilisés conformément à la présente invention, on peut citer à titre non limitatif :
- les silicones volatiles: celles-ci possèdent un point d'ébullition compris entre 60°C et 260°C. Elles sont choisies parmi les silicones cycliques contenant de 3 à 7 atomes de silicium et de préférence 4 à 5.
- les silicones non volatiles : elles sont constituées principalement par les polyalkylsiloxanes, les polyarylsiloxanes, les polyalkylarylsiloxanes, les gommes de silicone, les résines de silicone ou leurs mélanges et les silicones organomodifiées.

On utilise plus particulièrement les polydiméthylsiloxanes, les silicones aminées et les silicones oxyalkylénées.

Les silicones (polyorgano-siloxanes) peuvent être utilisées dans les compositions de l'invention dans des proportions comprises entre 0,01 et 20% en poids et de préférence entre 0,1 et 10% en poids par rapport au poids total de la composition.

Les compositions de l'invention peuvent contenir en outre un polymère cationique ou amphotère.

Les polymères cationiques utilisés ont généralement une masse moléculaire en nombre comprise entre 500 et 5.10⁶ environ, et de préférence comprise entre 10³ et 3.10⁶ environ.

Parmi les polymères cationiques, on peut citer les protéines (ou hydrolysats de protéines) quaternisées et les polymères du type polyamine, polyaminoamide et polyammonium quaternaire. Ce sont des produits connus.

Les protéines ou hydrolysats de protéines quaternisés sont en particulier des polypeptides modifiés chimiquement portant en bout de chaîne, ou greffés sur celles-ci, des groupements ammonium quaternaire. Leur masse moléculaire peut varier par exemple de 1 500 à 10 000, et en particulier de 2 000 à 5 000 environ.

Les polymères du type polyamine, polyaminoamide, polyammonium quaternaire, utilisables conformément à la présente invention, pouvant être notamment mentionnés, sont ceux décrits dans les brevets français n°2 505 348 ou 2 542 997. Parmi ces polymères, on peut citer:
(1) Les copolymères vinylpyrrolidone-acrylate ou -méthacrylate de dialkylaminoalkyle quaternisés ou non, tels que les polymères décrits en détail dans les brevets français 2 077 143 et 2 393 573.
(2) Les dérivés d'éthers de cellulose comportant des groupements ammonium quaternaire décrits dans le brevet français 1 492 597.
(3) Les dérivés de cellulose cationiques, tels que les copolymères de cellulose ou les dérivés de cellulose greffés avec un monomère hydrosoluble d'ammonium quaternaire, et décrits notamment dans le brevet US 4131 576, tels que les hydroxyalkyl celluloses, comme les hydroxyméthyl-, hydroxyéthyl- ou hydroxypropyl celluloses greffées, notamment avec un sel de méthacryloyléthyl triméthyl ammonium, de méthacrylamidopropyl triméthyl ammonium ou de diméthyl-diallylammonium.
(4) Les polysaccharides non cellulosiques et notamment gommes de guar cationiques décrits plus particulièrement dans les brevets US 3 589 578 et 4 031 307.
(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(6) Les polyaminoamides solubles dans l'eau préparés en particulier par polycondensation d'un composé acide avec une polyamine; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé, l'agent réticulant étant utilisé dans des proportions allant de 0,025 à 0,35 mole par groupement amine du polyaminoamide; ces polyaminoamides peuvent être alcoylés ou s'ils comportent une ou plusieurs fonctions amines tertiaires, quaternisés. De tels polymères sont notamment décrits dans les brevets français 2.252.840 et 2.368.508.
(7) Les dérivés de polyaminoamides résultant de la condensation de polyalcoylène polyamines avec des acides polycarboxyliques suivie d'une alcoylation par des agents bifonctionnels. On peut citer, par exemple, les polymères acide adipique diacoylamino-hydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone, le rapport molaire entre la polyalkylène polyamine et l'acide dicarboxylique étant compris entre 0,8: 1 et 1,4: 1, le polyaminoamide en résultant étant amené à réagir avec l'épichlorhydrine dans un rapport molaire d'épichlorhydrine par rapport au groupement amine secondaire du polyaminoamide compris entre 0,5: 1 et 1,8: 1. De tels polymères sont notamment décrits dans les brevets américains 3.227.615 et 2.961.347.
(8) Les cyclopolymères de méthyl diallyl amine ou de diméthyl diallyl ammonium, notamment ceux décrits dans le brevet français 2.080.759 et dans son certificat d'addition 2.190.406.
(9) Les polymères de diammonium quaternaire décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
(10) Les polymères de polyammonium quaternaire notamment décrits dans la demande de brevet EP-A-122 324.
(11) Les homopolymères ou copolymères dérivés des acides acrylique ou méthacrylique et comportant des motifs CH₂-CHRₐ-CO-O-A₁-NRₑR_{f} , CH₂-CHRₐ-CO-O-A₁-N⁺R_{b}R_{c}R_{d} , X⁻ et/ou CH₂-CHRₐ-CO-NH-A₁-N⁺R_{b}R_{c}R_{d} , X⁻ dans lesquels les groupements Rₐ désignent indépendamment H ou CH₃, les groupements A₁ désignent indépendamment un groupe alcoyle linéaire ou ramifié de 1 à 6 atomes de carbone ou un groupe hydroxyalcoyle de 1 à 4 atomes de carbone, les groupements R_{b}, R_{c}, R_{d}, identiques ou différents, désignent indépendamment un groupe alcoyle de 1 à 18 atomes de carbone ou un radical benzyle, les groupements Rₑ et R_{f} représentent un atome d'hydrogène ou un groupement alcoyle de 1 à 6 atomes de carbone, X⁻ désigne un anion, par exemple méthosulfate ou halogénure, tel que chlorure ou bromure,
(12) Les polymères quaternaires de vinylpyrrolidone et de vinylimidazole, tels que, par exemple, les produits commercialisés sous les dénominations "LUVIQUAT FC 905", "LUVIQUAT FC 550" et "LUVIQUAT FC 370" par la société BASF.
(13) Les polyamines comme le "POLYQUART H" vendu par HENKEL, référencé sous le nom de " POLYETHYLENEGLYCOL TALLOW POLYAMINE " dans le dictionnaire CTFA.
(14) Les polymères réticulés de sel de méthacryloyloxyéthyl triméthylammonium, tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléhnique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère les dérivés d'éthers de cellulose comportant des groupements ammonium quaternaire, les polysaccharides non cellulosiques et, notamment les gommes de guar cationiques et les cyclopolymères de méthyl diallyl ammonium ou de diméthyl diallyl ammonium.

Les polymères cationiques sont utilisés dans les compositions de l'invention dans des proportions comprises entre 0,001 et 20% en poids et de préférence entre 0,01 et 5% en poids par rapport au poids total de la composition.

Les compositions, selon l'invention, présentent un pH généralement compris entre 3 et 12, et plus particulièrement entre 4 et 8.

Le milieu cosmétiquement acceptable des compositions est constitué soit par de l'eau, soit par un mélange d'eau et d'au moins un solvant choisi parmi les alcools inférieurs, les alkylèneglycols et les éthers de polyols.

Les compositions selon l'invention peuvent contenir également des agents régulateurs de viscosité, tels que des électrolytes comme le chlorure de sodium, des épaississants comme les dérivés de la cellulose, tels que par exemple la carboxyméthylcellulose, l'hydroxypropyl- cellulose, l'hydroxyéthylcellulose, la gomme de guar, les gommes de guar hydroxypropylées, les scléroglucanes, la gomme de xanthane, les polymères amphiphiles comportant au moins une chaîne grasse.

Les compositions selon l'invention peuvent également contenir différents adjuvants habituellement utilisés en cosmétique, tels que des parfums, des conservateurs, des synergistes de mousse, des séquestrants, des stabilisateurs de mousse, des agents propulseurs, des colorants, des agents anti-pelliculaires, des céramides, des vitamines ou provitamines, des hydroxy acides, des agents acidifiants ou alcalinisants, des huiles végétales minérales, animales, des huiles de synthèse telles que les polyisobutènes et les polydécènes, des esters d'acides gras, des pseudocéramides, des agents nacrants ou d'autres adjuvants selon l'usage envisagé.

Les compositions conformes à l'invention peuvent également contenir jusqu'à 5 % d'agents nacrants ou opacifiants bien connus dans l'état de la technique tels que par exemple les oxydes de titane enrobés ou non, les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses ( par exemple en C8-C30) tels que par exemple le distéaryléther ou le 1-(hexadécyloxy)-2-octadécanol, les cyclodextrines, les alcools gras tels que les alcools cétylique, stéarylique et béhénylique.

L'invention a aussi pour objet l'utilisation comme shampooing de la composition sus définie.

Un autre objet de la présente invention est un procédé de lavage des matières kératiniques, qui consiste dans l'application sur ces matières d'au moins une composition de la présente invention, suivie d'un rinçage des matières traitées après un éventuel temps de pose.

Les exemples qui suivent sont destinés à illustrer l'invention sans pour autant présenter un caractère limitatif.

### EXEMPLE 1:

On a préparé les compositions de shampooing suivantes:

| Composition | Invention (A) | Comparatif (B) |
|---|---|---|
| Lauryléthersulfate de sodium (C12/C14 à 70/30) à 2.2 moles d'oxyde d'éthylène | 10 g M.A. | 10 g M.A. |
| Cocoyl amphohydroxypropyl sulfonate de sodium, en solution aqueuse à 32% de matière active, proposé sous la dénomination Miranol CSE par la société RHODIA | 3.2 g M.A. | - |
| Cocoamidopropyl bétaïne | - | 3.2 g M.A. |
| Hydroxyéthylcellulose réticulée à l'épichlorhydrine, quaternisée par la triméthylamine, vendue sous la dénomination JR400 par la société AMERCHOL | 0.3 g | 0.3 g |
| Monoisopropanolamide d'acides de coprah | 2.5 g | 2.5 g |
| Conservateurs | q.s. | q.s. |
| Acide citrique ou soude q.s. | pH 7 | pH 7 |
| Eau déminéralisée q.s.p. | 100 g | 100 g |

On effectue un shampooing en appliquant environ 6 g de la composition A sur une demi-tête de cheveux naturels préalablement mouillés. On fait mousser le shampooing puis on rince abondamment à l'eau. On sèche les cheveux avec un sèche-cheveux.

On procède selon le même mode opératoire que ci-dessus avec la composition comparative B sur l'autre demi-tête.

Les experts comparent les demi-têtes deux à deux.

Un panel d'experts a évalué l'aspect des cheveux séchés et a noté un plus grand lissage au toucher avec la composition conforme à l'invention.

Les cheveux traités avec la composition A sont significativement plus lisses que ceux traités avec la composition B.

### EXEMPLE 2:

On a préparé les compositions de shampooing suivantes:

| Composition | Invention (C) | Comparatif (D) |
|---|---|---|
| Lauryléthersulfate de sodium (C12/C14 à 70/30) à 2.2 moles d'oxyde d'éthylène | 14 g M.A. | 14 g M.A. |
| Cocoyl amphohydroxypropyl sulfonate de sodium, en solution aqueuse à 32% de matière active, proposé sous la dénomination Miranol CSE par la société RHODIA | 3 g M.A. | - |
| Cocoamidoéthyl (N-hydroxyéthyl, N-carboxyméthyl) glycinate de sodium, vendu sous la dénomination Miranol C2M par la société RHODIA | - | 3 g M.A. |
| Gomme de guar modifiée par du chlorure de 2,3-époxypropyl triméthyl ammonium vendue sous la dénomination Jaguar C13 S par la société RHODIA | 0.18 g | 0.18 g |
| Polydiméthylsiloxane de viscosité 60 000 cSt, vendue sous la dénomination DC 200 fluid 60000 par la société DOW CORNING | 2.5 g | 2.5 g |
| Mélange de 1-hexadécyloxyoctadodécanol et d'alcool cétylique Conservateurs | 2.5 g | 2.5 g |
| | q.s. | q.s. |
| Parfum | q.s. | q.s. |
| Acide citrique ou soude (NaOH) q.s. | pH 7.5 | pH 7.5 |
| Eau déminéralisée q.s.p. | 100 g | 100 g |

On effectue un shampooing en appliquant environ 6 g de la composition A sur une demi-tête de cheveux naturels préalablement mouillés. On fait mousser le shampooing puis on rince abondamment à l'eau. On sèche les cheveux avec un sèche-cheveux.

On procède selon le même mode opératoire que ci-dessus avec la composition comparative B sur l'autre demi-tête.

Les experts comparent les demi-têtes deux à deux.

Un panel d'experts a évalué l'aspect des cheveux séchés et a noté plus de gonflant, de plis et de lissage au toucher.

### EXEMPLES 3 ET 4

On a préparé les compositions suivantes :

| Composition | Exemple 3 | Exemple 4 |
|---|---|---|
| Lauryléthersulfate de sodium (C12/C14 à 70/30) à 2.2 moles d'oxyde d'éthylène | 11.2 g M.A. | 16 g M.A. |
| Cocoyl amphohydroxypropyl sulfonate de sodium, en solution aqueuse à 32% de matière active, proposé sous la dénomination Miranol CSE par la société RHODIA | 3 g M.A. | 2.7 g M.A. |
| Hydroxyéthylcellulose réticulée par l'épichlorhydrine et quaternisée par la triméthylamine, vendue sous la dénomination JR 400 par la société AMERCHOL | 0.8 g | - |
| Gomme de guar modifiée par du chlorure de 2,3-époxypropyl triméthyl ammonium vendue sous la dénomination Jaguar C13 S par la société RHODIA | - | 0.4 g |
| Distéarate de glycol | 2 g | - |
| Distéaryl éther | - | 1.5 g |
| Alcool béhénylique | - | 1.5 g |
| Monoisopropanolamide d'acides de coprah | 2.2 g | - |
| Acide polyacrylique réticulé | 0.2 g | 0.2 g |
| Chlorure de sodium | - | 1 g |
| Acide citrique | 3 g | - |
| Conservateurs | q.s. | q.s. |
| Parfum | q.s. | q.s. |
| Acide citrique ou soude q.s | - | pH 7.5 |
| Ammoniaque q.s. | pH 5.3 | - |
| Eau déminéralisée q.s.p. | 100 g | 100 g |

Les cheveux lavés avec ces compositions sont lisses et facile à démêler.

### EXEMPLES 5 ET 6

On a préparé les compositions de shampooing suivantes :

| Composition | Exemple 5 | Exemple 6 |
|---|---|---|
| Lauryléthersulfate de sodium (C12/C14 à 70/30) à 3 moles d'oxyde d'éthylène | 6.3 g M.A. | - |
| Lauryléthersulfate de sodium (C12/C14 à 70/30) à 2.2 moles d'oxyde d'éthylène | 1.5 g M.A. | 1.1 g M.A. |
| Lauryléthersulfate d'ammonium | - | 8.5 g M.A. |
| Cocoyl amphohydroxypropyl sulfonate de sodium, en solution aqueuse à 38% de matière active, commercialisé sous la dénomination Miranol CS par la société RHODIA | 3.0 g M.A. | 3.2 g M.A. |
| Cocoamidoéthyl (N-hydroxyéthyl, N-carboxyméthyl) glycinate de sodium, (Miranol C2M par la société RHODIA) | 0.8 g M.A. | 0.5 g M.A. |
| Hydroxyéthylcellulose réticulée par l'épichlorhydrine et quaternisée par la triméthylamine, vendue sous la dénomination JR 400 par la société AMERCHOL | 0.3 g | 0.4 g |
| Monoéthanolamide d'acides de coprah | 0.8 g | - |
| Distéarate d'éthylène glycol | 2 g | 1.5 g |
| Acide polyacrylique réticulé | 0.2 g | 0.2 g |
| Conservateurs | q.s. | q.s. |
| Parfum | q.s. | q.s. |
| Acide citrique | q.s.. pH 6.7 | 3 g |
| Ammoniaque | - | q.s.. pH 5.3 |
| Eau déminéralisée q.s.p. | 100 g | 100 g |

## Revendications

1. Composition de lavage des matières kératiniques, **caractérisée par le fait qu'**elle comprend, dans un milieu aqueux cosmétiquement acceptable comprenant au moins un tensio-actif anionique alkyléthersulfate et au moins 2,5% d'un tensioactif amphotère alcoylamphohydroxyalkylsulfonate, le rapport en poids tensioactif alkyléthersulfate / tensioactif amphotère sulfonate allant de 2 à 20.

2. Composition selon la revendication 1, **caractérisée en ce que** les tensioactifs anioniques alkyléthersulfates sont les sels des alkyléther sulfates, des alkylamidoéther sulfates, des alkylaryléther sulfates; le radical alkyle de tous ces différents composés comportant de préférence de 8 à 24 atomes de carbone, et le radical aryle désignant de préférence un groupement phényle ou benzyle.

3. Composition selon l'une quelconque des revendications précédentes, **caractérisée en ce que** le tensioactif anionique de type alkyléthersulfate est choisi parmi les sels d'alkyléthersulfates en C₁₀-C₁₄ et plus particulièrement ceux en C₁₂-C₁₄.

4. Composition selon l'une quelconque des revendications 1 à 3, **caractérisée par le fait que** ledit tensioactif amphotère est choisi parmi les composés alcoylamphohydroxyalkylsulfonates ayant la formule suivante (I) : 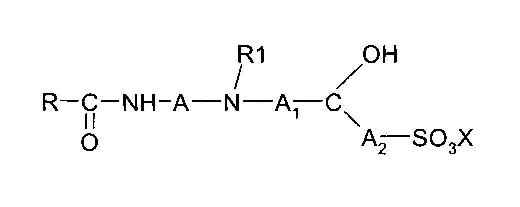 dans laquelle :
R désigne un radical hydrocarboné, saturé ou insaturé, linéaire ou ramifié comportant de 5 à 29 atomes de carbone.
R1 désigne un radical hydroxyalkyle en C₁-C₄, de préférence hydroxyéthyle,
A, A1, A2 , identiques ou différents, désignent un radical alkylène divalent linéaire ou ramifié en C₁-C₁₀, de préférence en C₁-C₃
X désigne un atome d'hydrogène, un cation minéral ou organique.

5. Composition selon la revendication 4, **caractérisée par le fait que** R désigne de préférence un radical alkyle ou alkényle mono ou polyinsaturé comportant de 5 à 29 atomes de carbone et de préférence de 7 à 22 atomes de carbone et encore plus particulièrement de 9 à 17 atomes de carbone.

6. Composition selon l'une quelconque des revendications 4 ou 5, **caractérisée par le fait que** A et A2 désignent -CH₂CH₂-.

7. Composition selon l'une quelconque des revendications 4 à 6, **caractérisée par le fait que** A1 désigne -CH₂-.

8. Composition selon l'une quelconque des revendications 4 à 7, **caractérisée par le fait que** la cation minéral ou organique est choisi parmi celui d'un métal alcalin ( par exemple Na⁺, K⁺) , celui d'un métal alcalinoterreux (Mg²⁺, Ca²⁺) , l'ion NH₄⁺, les ions ammoniums issus des aminoacides basiques ou des amino-alcools.

9. Composition selon l'une quelconque des revendications 1 à 8, **caractérisée par le fait que** ledit tensioactif amphotère est choisi parmi les sels de Cocoyl amphohydroxypropyl sulfonate et les sels de palmitoyl amphohydroxypropyl sulfonate.

10. Composition selon l'une quelconque des revendications précédentes, **caractérisée en ce que** ledit tensioactif anionique alkyléthersulfate est présent dans des proportions comprises entre 2,5 et 50% en poids par rapport au poids total de la composition, et de préférence entre 5 et 40% en poids.

11. Composition selon l'une quelconque des revendications précédentes, **caractérisée en ce que** ledit tensioactif amphotère alcoylamphohydroxyalkylsulfonate est présent dans des proportions comprises entre 2,5% et 30% en poids par rapport au poids total de la composition, et de préférence entre 3 et 15% en poids.

12. Composition selon l'une quelconque des revendications précédentes, **caractérisée en ce que** le rapport en poids tensioactif alkyléthersulfate / tensioactif amphotère sulfonate va de 2 à 10.

13. Composition selon l'une quelconque des revendications précédentes, **caractérisée par le fait qu'**elle contient en outre au moins un tensioactif choisi parmi les tensio-actifs non-ioniques, amphotères ou zwitterioniques différents des tensioactifs amphotères sus-définis.

14. Composition selon la revendication 13, **caractérisée en ce que** le ou les tensioactifs additionnels sont présents dans des quantités allant de 0,5 à 15% en poids.

15. Composition selon l'une quelconque des revendications précédentes, **caractérisée en ce que** la quantité totale de tensioactifs va de 5 à 50% en poids et de préférence de 7 à 30% en poids par rapport au poids total de la composition.

16. Composition selon l'une quelconque des revendications précédentes, **caractérisée par le fait qu'**elle contient en outre un ou plusieurs agents tensio-actifs cationiques.

17. Composition selon l'une quelconque des revendications précédentes, **caractérisée par le fait qu'**elle contient en outre une ou plusieurs silicones.

18. Composition selon l'une quelconque des revendications précédentes, **caractérisée par le fait qu'**elle contient en outre un ou plusieurs polymères cationiques ou amphotères.

19. Composition selon la revendication 18, **caractérisée par le fait qu'**elle contient au moins un polymère cationique choisi parmi les dérivés d'éthers de cellulose comportant des groupements ammonium quaternaire, les polysaccharides non cellulosiques, notamment les gommes de guar cationiques et les cyclopolymères de méthyl diallyl ammonium ou de diméthyl diallyl ammonium.

20. Composition selon l'une quelconque des revendications précédentes, **caractérisée par le fait qu'**elle contient en outre des agents régulateurs de viscosité, tels que des électrolytes comme le chlorure de sodium, des épaississants comme les dérivés de la cellulose, la gomme de guar, les gommes de guar hydroxypropylées, les scléroglucanes, la gomme de xanthane et les polymères amphiphiles comportant au moins une chaîne grasse.

21. Composition selon l'une quelconque des revendications 1 à 20, **caractérisée par le fait qu'**elle contient en outre un ou plusieurs adjuvants choisis parmi les parfums, les conservateurs, les synergistes de mousse, les séquestrants, les stabilisateurs de mousse, les agents propulseurs, les colorants, les agents anti-pelliculaires, les céramides, les vitamines ou provitamines, les hydroxy-acides, les agents acidifiants ou alcalinisants, les huiles végétales minérales, animales, les huiles de synthèse telles que les polyisobutènes et les polydécènes, les esters d'acides gras, les pseudocéramides et les agents nacrants et/ou opacifiants.

22. Composition selon la revendication 21, **caractérisée par le fait que** d'agents nacrants et/ou opacifiants sont choisis parmi les oxydes de titane enrobés ou non, les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses ( par exemple en C8-C30) tels que par exemple le distéaryléther ou le 1-(hexadécyloxy)-2-octadécanol, les cyclodextrines, les alcools gras tels que les alcools cétylique, stéarylique et béhénylique.

23. Utilisation d'au moins 2,5% d'un tensioactif amphotère alcoylamphohydroxyalkylsulfonate dans le but d'améliorer le démêlage et le lissage des compositions de lavage des matières kératiniques contenant un tensio-actif anionique alkyléthersulfate.

24. Utilisation comme shampooing de la composition telle que définie dans l'une quelconque des revendications 1 à 22.

25. Procédé de lavage des matières kératiniques, **caractérisé par** l'application sur ces matières d'au moins une composition telle que définie dans l'une quelconque des revendications 1 à 22, suivie d'un rinçage des matières traitées après un éventuel temps de pose.
